# EUROPEAN PATENT APPLICATION

(11) **EP 0 997 199 A1**
(43) Date of publication of application: **03.05.2000**
(21) Application number: 98203633.7
(22) Date of filing: 26.10.1998
(51) Int. Cl.: B03D 1/02, C07D 499/18, C07D 501/12, C12P 35/04, C12P 37/04

(54) **Method for separation of solid compounds in suspension**

(71) Applicant: DSM N.V., 6411 TE Heerlen (NL)
(72) Inventor: De Vroom, Erik, 2313 JM Leiden (NL); Kers, Ernst Edmund, 3031 PM Rotterdam (NL); Heijnen, Joseph Johannes, 5121 NR Rijen (NL)
(74) Representative: Visser-Luirink, Gesina, Dr.

(57) **Abstract**

A new method for separation of a partial suspension of a mixture of solid compounds into two or more single solid compounds and a mother liquor by the application of flotation has been described.

## Description

### Field of the invention

The present invention relates to a new method for the separation of one or more single solid compounds from a partial suspension of solid compounds by means of flotation.

### Background of the invention

The most commonly used methods for the separation of organic end products from a mixture of solid compounds like starting materials and intermediates in a reaction mixture are either fractional crystallization, extraction or chromatography. However, these methods quite often lead to loss of valuable materials and overall higher costs of production thereof.

One of the less known techniques for selective separation of solids compounds is by means of flotation. The flotation technique is usually applied for metal ores concentration and in waste water treatment. The German patent document DE 4105384 mentions the selective flotation of phosphorus minerals from ores deposits by the addition of N-acylated protein, peptide or penicillin hydrolysate as the flotation reagent.

Another German patent document DE 2621349 describes the separation technique of flotation by adding a flotation reagent and/or a gaseous medium to the mixture such as sludge, produced in municipal sewage or industrial waste treatment plants.

The object of the present invention is to provide a simple and widely applicable method, enabling organic compounds to be isolated from a mixture of solid compounds in pure form and without large loss of valuable materials.

Surprisingly, it has been found that this can be achieved by subjecting a suspension or a partial suspension of the worked-up end product in a reaction mixture to flotation.

The method according to the present invention can suitably be applied for the separation of solid compounds used for medical purposes into two or more single solid compounds and a mother liquor such as for the production of β-lactam antibiotics.

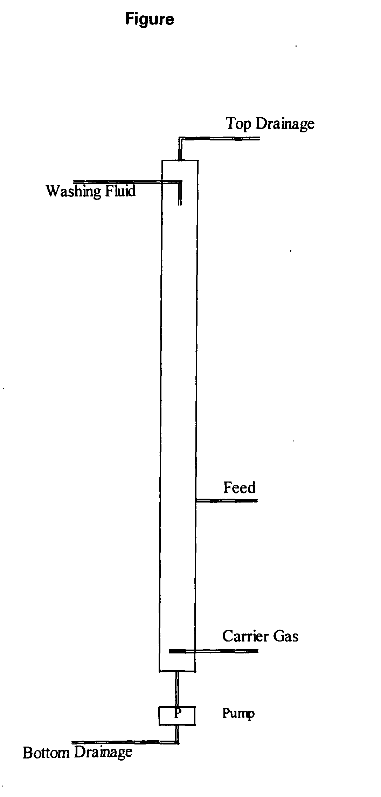

### Description of the Figure

A vertical column is fitted at the bottom with a Drainage outlet for pumping out the suspension of the products and at the top a Draining outlet for removing the foam generated during the flotation process. A partial suspension of solid compounds to be separated is inserted into the column *via* the Feed inlet. The gaseous phase is introduced into the column near to the bottom of the column *via* the Carrier Gas inlet and the washing liquid is passed through the Washing Fluid inlet.

### Description of the invention

The present invention describes a method for separation of a partial suspension of solid compounds into two or more single solid compounds and a mother liquor by the application of flotation on the suspension of a mixture of compounds in water and/or one or more organic solvents. The partial suspension of solid compounds refers to a suspension in water and/or organic solvent of solid compounds in which a part of the same is in a soluble state and the rest in a solid state. The solid compounds to be separated can be produced by a chemical process and/or an enzymatic process. The process of flotation can be applied in a separation vessel, separation funnel or column, preferably in a column.

The flotation is achieved by one or more of the measures selected from shaking, stirring and introducing a gaseous phase into the suspension. Preferably the gaseous phase is compressed air or nitrogen. The gaseous phase can be introduced into the lower 40% part of the column, preferably at the bottom of the same. Preferably the mother liquor is recirculated into the suspension of the mixture of compounds.

For the sake of illustration, the process of flotation in a column is depicted in the figure enclosed.

The compounds which can be separated from the mixture of compounds suspended in one or more solvents are organic compounds, preferably a β-lactam compound with the general formula (I): with 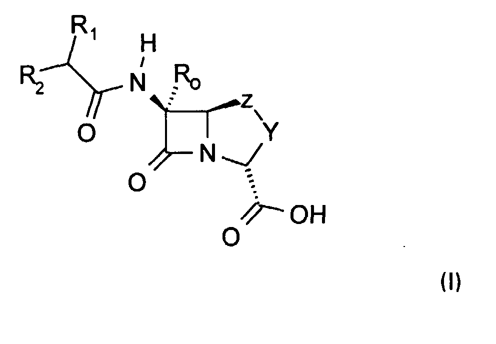
R₀ is hydrogen or C₁₋₃ alkoxy;
R₁ is hydrogen, hydroxy, amine, halogen or lower alkyl;
R₂ is an optionally substituted phenyl or phenoxy or 5- or 6-membered heterocyclic ring;
Z is oxygen, sulphur or an oxidized form of sulphur or CH₂ or optionally substituted CH₂; and
Y is 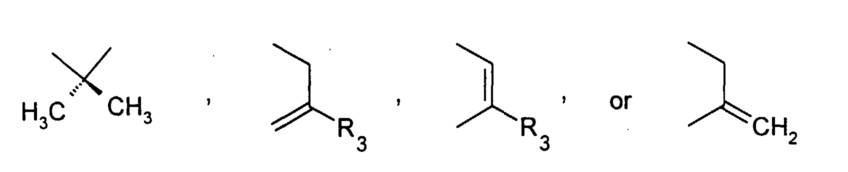 wherein
R₃ is hydrogen, hydroxy, halogen, C₁₋₃alkoxy, optionally substituted, optionally containing one or more heteroatoms, saturated or unsaturated, branched or straight C₁₋₅alkyl, preferably methyl, optionally substituted, optionally containing one or more heteroatoms, C₅₋₈cycloalkyl, optionally substituted aryl or heteroaryl, or optionally substituted benzyl.

For the preparation of β-lactam antibiotics, a β-lactam nucleus such as 6-aminopenicillanic acid (6-APA), 7-aminocephalosporanic acid (7-ACA), 7-amino-3-chloro-3-(desacetoxymethyl)cephalosporanic acid (7-ACCA), 7-amino-3'-desacetylcephalosporanic acid (7-ADAC), or 7-amino-3'-desacetoxycephalosporanic acid (7-ADCA) is acylated with an amino acid such as D-(-)-phenylglycine (PG), D-(-)-4-hydroxyphenylglycine (HPG) or (-)-4-hydroxyphenylglycine methyl ester (HPGM). The acylation can be performed by means of chemical activation such as described in European patent application EP 011513, United States patent US 4,248,780, German patent applications DE 1302847, DE 2020133, DE 2065879 and British Patents GB 1327270 and GB 1347979. The acylation can also be done by using enzymatic catalysis such as described in European patent application EP 730036 and International patent application WO 96/23897. An activated side chain of formula (II) can be used for the enzymatic acylation of a β-lactam nucleus: 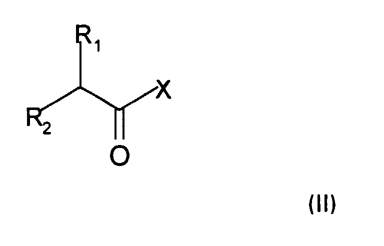 with
R₁, R₂ is defined as above, X is NHR₃, wherein R₃ is hydrogen or lower alkyl or X is alkoxy, preferably lower alkoxy and more preferably methoxy.

Example of the kinetically controlled enzymatic acylation with an activated derivative of the side chain such as an amide of PG (for example D-(-)-phenylglycine amide, PGA), or an ester of PG (for example D-(-)-phenylglycinemethyl ester, PGM) is described in International Patent Application WO 92/01061, wherein the hydrolysis of the acylating agent and the β-lactam antibiotic results in the formation of PG. Apart from the β-lactam antibiotic to be produced and PG, the mixtures obtained from an enzymatic condensation may also contain the unreacted β-lactam nucleus and the unreacted acylating agent such as PGA or PGM. However, the exact composition of the mixture obtained is not critical when subjected to the procedure of the present invention. Mixtures that are suitable for purification according to the, present invention are mixtures that contain 10-1500 mM (preferably 50-1000 mM) β-lactam antibiotic, 20-1500 mM (preferably 50-1000 mM) hydrolyzed side chain, 0-1000 mM (preferably 0-200 mM) β-lactam nucleus, and 0-1000 mM (preferably 0-400 mM) activated the side chain. The main problem of β-lactam production processes is the recovery of product from the complex reaction mixtures. In many cases, downstream processing is hampered by losses of product resulting from degradation and mother liquor disposal.

The advantage of the present invention is that a β-lactam antibiotic can be recovered in a mild, simple and industrially feasible manner. The method not only minimizes losses in product, but also offers a new method for the recovery of a major side product formed during the enzymatic production of Ampicillin, Cefaclor and Cephalexin, namely D-(-)-phenylglycine (PG), or in the enzymatic production of Amoxicillin, Cefadroxil, Cefatrizine and Cefprozil, namely D-(-)-4-hydroxyphenylglycine (HPG), or during the enzymatic production of Cefroxadine, Cephradine and Epicillin, namely D-(-)-dihydrophenylglycine. After the reaction is terminated when the maximal conversion is reached, the reaction mixture is usually present in the form of a suspension of several solid components such as the antibiotic formed, the unreacted starting materials PG or HPG and immobilized enzyme. Preferably, the immobilized enzyme is recovered. A suitable method is filtration of the condensation mixture over a sieve that will retain the immobilized enzyme. In the next step, the valuable components such as the antibiotic end product and PG or HPG are recovered according to the present invention wherein a separation is obtained by means of flotation.

Preferably the β-lactam antibiotic is chosen from the group consisting of ampicillin, amoxicillin, epicillin, cephalexin, cefaclor, cefloglycin, cefadroxil, cefatrizine, cefradrin, cefroxadine, cefprozil and loracarbef.

The invention can also be used together with well-known separation techniques such as filtration, to improve a separation process of a compound from a suspension of solid compounds.

The invention will now be described with reference to the following Examples, which are provided for illustrative purposes only.

### Examples

### Definitions

Assemblase® is an immobilized E. Coli penicillin acylase from ATCC 1105, as described in the International Patent Application WO 97/04086. The immobilization is carried out as described in European Patent Application EP 222462, using gelatin and chitosan as gelating agent and glutaraldehyde as crosslinking agent. The activity of the Assemblase® depends on the amount of enzyme attached to the beads and is, in the examples given below, 3 ASU.g-1 dry weight (1 ASU being defined as the amount of enzyme that produces 1 g Amoxicilline.3H₂O per hour from 6-APA and HPGM at 20°C).

### Example 1

### Separation of ampicillin and phenyl glycine from a partial suspension resulting from the enzymatic acylation reaction in which the suspension from the bottom exit of the column resulting during the flotation is recirculated back into the buffer vessel.

### STEP A: Preparation of a solution of PGA(H₂SO₄)_{1/2}

301.6 g PGA (2.00 mol) is suspended in 650 g water at 5°C. 102.1 g 96% H₂SO₄ (1.00 mol) is added under stirring in one hour. During this process, the temperature is maintained below 25°C.

### STEP B: Enzymatic synthesis of Ampicillin

An enzymatic reactor (1.5 l vessel, diameter 0.11 m, equipped with a 175 µm mesh sieve-bottom) is charged with 300 g Assemblas® (obtained by separating the immobilized enzyme from an enzyme slurry, washing the beads with water, and weighing the beads without drying). In a second vessel (1.2 l) 131.6 g 6-APA (0.600 mol), 30.2 g PGA (0.200 mol) and 400 ml water is stirred for 1 5 mm at 10°C) and then transferred to the enzyme reactor using 100 ml water as transferring fluid. At 10°C, the enzyme reactor is supplied with 423.7 g PGA(H₂SO₄)_{1/2}-solution from STEP A (0.800 mol) during 233 min using a constant speed in such a way that the pH is maintained at 6.3. After 295 min, the pH is maintained at 6.3 by means of titrating with a 12 M solution of H₂SO₄. After 570 mm the pH is lowered to 5.0 using a 12 M solution of H₂SO₄. The enzyme reactor now contains 575 mmol Ampicillin, 15 mmol 6-APA, 50 mmol PGA and 365 mmol PG.

### STEP C: Removal of the Ampicillin/PG slurry from the enzyme reactor

The slurry of Ampicillin and PG as obtained in STEP C is removed from the enzyme reactor using filtration in combination with stirring at 500 rpm. The reactor is rinsed with 10 x 250 ml water (10° C). The resulting white slurry contains > 99.8% of the Ampicillin produced and > 99.5% of the PG produced. The Assemblase® is retained in the enzyme reactor for > 99.5%.

### STEP D: Separation of Ampicillin and PG using flotation in a column

In this example a glass column is used with a length of 230 cm and an internal diameter of 2 cm.

The column (length = 230 cm and internal diameter = 2 cm) is fed (flow 1.5 l/h) from a buffer vessel at a height of 96 cm with a suspension of STEP C. Through the column air is blown with a flow of 2 l/h. The suspension from the bottom exit is passed back into the buffer vessel. The foam that is formed on the top of the column is removed from the system. After 18 hours the composition of the solid material in the buffer vessel has changed from 65% Ampicillin and 16% PG to 86% Ampicillin and 4% PG. At that point, the material leaving the bottom exit contains 86% Ampicillin and 0% PG.

### Example 2

### Separation of ampicillin and phenyl glycine from a partial suspension resulting from the enzymatic acylation reaction in which the suspension from the bottom exit and the foam at the top of the column resulting during the flotation is recirculated back into the buffer vessel.

### Separation of Ampicillin and PG using flotation in a column

The same column is used as in Example 1. The column is fed from a buffer vessel at a height of 96 cm with a suspension produced in a similar method as described in the Example 1. Through the column air is blown with a flow of 2 l/h. The suspension which is drained from the column at the bottom (flow 300 ml/h) is continuously filtrated and the mother liquor is returned to the top of the column. The foam that is formed on the top of the column is continuously passed back to the buffer vessel. The solid material in the suspension in the buffer vessel had at the start a composition of 69% Ampicillin and 18% PG. The crystal cake isolated from the bottom of the column contains 85% Ampicillin and 0% PG. The foam formed in the top contains 53% Ampicillin and 40% PG.

## Claims

1. A method for separation of a suspension of solid compounds into two or more single solid compounds and a mother liquor, characterised by the application of flotation on the suspension of a mixture of compounds in water and/or one or more organic solvents.

2. A method according to claim 1, wherein the flotation is applied in a separation vessel, separation funnel or column.

3. A method according to claim 2, wherein preferably the flotation is applied in a column.

4. A method according to any one of the claims 1-3, wherein the flotation is achieved by one or more of the measures selected from shaking, stirring and introducing a gaseous phase into the suspension.

5. A method according to claim 4, wherein the gaseous phase is introduced into the lower 40% part of the column, preferably at the bottom of the same.

6. A method according to claim 5, wherein the gaseous phase is air or nitrogen.

7. A method according to any one of the claims 1-6, wherein the mother liquor is recirculated into the suspension of the mixture of compounds.

8. A method according to claims 1-7, wherein one of the compounds is an organic compound, preferably a β-lactam compound of general formula (I): 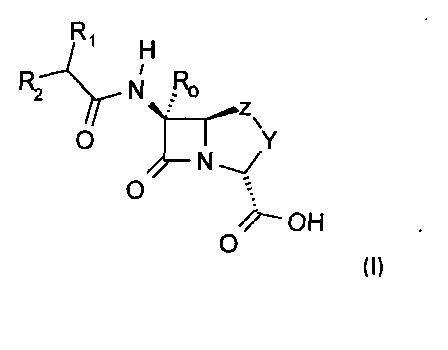 with
R₀ is hydrogen or C₁₋₃ alkoxy;
R₁ is hydrogen, hydroxy, amine, halogen or lower alkyl;
R₂ is an optionally substituted phenyl or phenoxy or 5- or 6-membered heterocyclic ring;
Z is oxygen, sulphur or an oxidized form of sulphur or CH₂ or optionally substituted CH₂; and
Y is 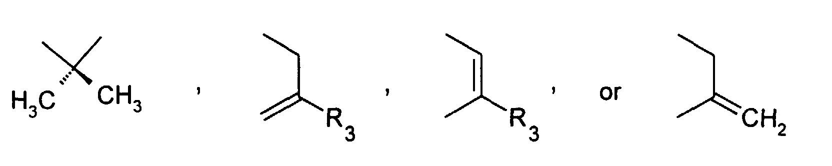 wherein
R₃ is hydrogen, hydroxy, halogen, C₁₋₃alkoxy, optionally substituted, optionally containing one or more heteroatoms, saturated or unsaturated, branched or straight C₁₋₅alkyl, preferably methyl, optionally substituted, optionally containing one or more heteroatoms, C₅₋₈cycloalkyl, optionally substituted aryl or heteroaryl, or optionally substituted benzyl.

9. A method according to claim 8, wherein the mixture of compounds containing the β-lactam compound is prepared by an enzymatic condensation reaction of a β-lactam nucleus with an activated side chain of formula (II): with 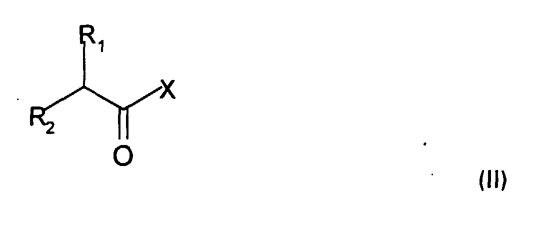
R₁, R₂ is defined as in claim 8, X is NHR₃, wherein R₃ is hydrogen or lower alkyl or X is alkoxy, preferably lower alkoxy and more preferably methoxy.

10. A method by using enzymatic condensation according to claim 9, wherein the mixture contains,
10-1500 mM, preferably 50-1000 mM of a β-lactam antibiotic,
0-1500 mM, preferably 50-1000 mM of hydrolyzed side chain,
0-1000 mM, preferably 0-200 mM of β-lactam nucleus, and
0-1000 mM, preferably 0-400 mM of activated side chain.

11. A method according to claims 6-8, wherein the β-lactam antibiotic is chosen from the group consisting of ampicillin, amoxicillin, epicillin, cephalexin, cefaclor, cefadroxil, cefradrin, cefroxadine, cefprozil and loracarbef.

12. Use of the flotation method to improve a separation process of a compound from a suspension of solid compounds.
